Europäisches Patentamt

European Patent Office

Office européen des brevets

(11) Veröffentlichungsnummer: **0 198 958**
**B1**

(12) **EUROPÄISCHE PATENTSCHRIFT**

(45) Veröffentlichungstag der Patentschrift: **29.08.90**

(21) Anmeldenummer: **85115874.1**

(22) Anmeldetag: **12.12.85**

(51) Int. Cl.⁵: **A 61 N 2/08,** A 61 F 13/02,
H 01 F 1/117

(54) **Dauermagnetische Anordung.**

(30) Priorität: **29.03.85 DE 3511395**

(43) Veröffentlichungstag der Anmeldung:
**29.10.86 Patentblatt 86/44**

(45) Bekanntmachung des Hinweises auf die
Patenterteilung:
**29.08.90 Patentblatt 90/35**

(84) Benannte Vertragsstaaten:
**AT CH DE FR GB IT LI NL SE**

(56) Entgegenhaltungen:
**WO-A-84/00305**
**AU-B- 424 257**
**DE-A-2 146 001**
**DE-A-3 227 506**
**DE-A-3 331 777**
**DE-A-3 334 699**
**DE-A-3 338 599**
**DE-B-1 943 914**

(73) Patentinhaber: **Rheinmagnet Horst Baermann
GmbH
Ohlenhohnstrasse 23
D-5206 Neunkirchen-S. 1 (DE)**

(72) Erfinder: **Baermann, Horst
Auf dem Saan 36
D-5064 Rösrath (DE)**

(74) Vertreter: **Dahlke, Werner, Dipl.-Ing. et al
Frankenforster Strasse 137
D-5060 Bergisch Gladbach 1 (DE)**

EP 0 198 958 B1

## Beschreibung

Die Erfindung bezieht sich auf eine dauermagnetische Anordnung, die aus einer oder mehreren gummiartig-flexiblen Magnetfolien oder- bändern mit darin eingebetteten hochkoerzitiven dauermagnetischen Teilchen besteht, die eine röhrenförmige Gestalt besitzt oder in eine derartige Form gebracht werden kann und die eine senkrecht zur Folien- bzw. Bandoberfläche verlaufende Magnetisierung aufweist.

Eine dauermagnetische Anordnung der genannten Art ist aus der WO—A—8 400 305 bekannt. Diese Anordnung besteht in einer flexiblen, tafelartigen Form mit darin eingebetteten magnetischen Teilchen, wobei eine Oberfläche der Form eine überwiegende Anzahl von in die Oberfläche eintretenden Feldlinien und die andere, gegenüberliegende Oberfläch eine überwiegende Anzahl von aus der Oberfläche heraustretenden Feldlinien aufweist. Die flexible Form ist zur therapeutischen Anwendung bestimmt und wird mit der Nordpolseite auf die Haut eines zu behandelnden Körperteils aufgelegt.

Um größere magnetische Felder im mittleren Bereich einer röhrenförmigen Anordnung zu erzeugen, sind auch schon mit einer Wicklung versehene Spulen in Form eines Tunnels zur Erzeugung elektromagnetischer Wechselfelder verwendet worden. Diese Geräte sind jedoch schwer und erlauben meist nur eine stationäre Anwendung. Darüber hinaus erfordern sie eine aufwendige Bauweise, so daß ihre Herstellungskosten hoch sind. Diese Nachteile weisen dauermagnetische Anordnungen röhrenförmiger Gestalt nicht auf.

Der Erfindung liegt die Aufgabe zugrunde, eine dauermagnetische Anordnung der eingangs genannten Art zu schaffen, mit der ein im mittleren Bereich weitgehend homogenes Magnetfeld mit hoher Feldstärke erzeugt werden kann.

Diese Aufgabe wird dadurch gelöst, daß die gegensätzlichen Magnetpole sich diametral oder axial in bezug auf die röhrenförmige Gestalt gegenüberstehen und der Gesamtinduktionsfluß des oder der Südpole an der einen, nach innen weisenden Seite der Anordnung im wesentlichen gleich dem Gesamtinduktionsfluß des oder der Nordpole an der anderen, nach innen weisenden Seite der Anordnung ist, und etwa eine homogene kraftliniendichte erzeugen.

Aufgrund der erfindungsgemäßen Konstruktion wird im mittleren Bereich des von den Magnetfolien oder -bändern gebildeten Tunnels eine hohe Feldliniendichte und die gewünschte weitgehende Homogenität der Feldlinien erreicht.

In einer bevorzugten Ausbildung der Erfindung weist die dauermagnetische Anordnung zwei sich diametral gegenüberstehende halbröhrenförmige Anordnungen auf, wobei jede Anordnung eine oder mehrere übereinander angeordnete Magnetfolien oder -bänder aufweist und wobei die nach innen weisenden Seiten der Magnetfolien oder -bänder der einen Anordnung eine bestimmte Polarität und die nach innen weisenden Seiten der anderen Anordnung die dazu entgegengesetzte Polarität aufweisen. Durch Übereinanderlagerung mehrerer Magnetfolien oder -bänder, wobei jeweils ein Nordpol der einen Folie dem Südpol der benachbart anliegenden Folie gegenüberliegt, wird die Feldliniendichte noch vergrößert. Durch diese Maßnahme kann man z.B. bei Verdoppelung der Foliendicke etwa die doppelte Flußdichte erhalten, ohne die Flexibilität wesentlich zu beeinträchtigen. Diese Ausführung ist bei Verwendung wohlfeiler Dauermagnetpulver besonders vorteilhaft.

In einer anderen bevorzugten Ausbildung der Erfindung weist die dauermagnetische Anordnung mindestens zwei sich axial gegenüberstehende röhrenförmige Bereiche mit abwechselnder Polfolge auf. Mit dieser Anordnung wird ein axiales, im mittleren Bereich der Anordnung weitgehend homogenes Magnetfeld mit der gewünschten Feldliniendichte erreicht.

Die sich axial gegenüberstehenden Bereiche können durch unterschiedliche Magnetisierungen einer oder mehrerer übereinander angeordneter Magnetfolien oder -bänder gebildet sein. Durch Verwendung mehrerer übereinander angeordneter Magnetfolien oder -bänder wird auch hier die Flußdichte noch vergrößert.

Die Bereiche können andererseits auch durch getrennt nebeneinander angeordnete Magnetfolien oder -bänder gebildet sein. Dabei werden die getrennt nebeneinander angeordneten Magnetfolien oder -bänder bevorzugt in einem starren Gehäuse angebracht.

Zum Anlegen des Gehäuses um einen Gegenstand, in dem ein magnetisches Feld erzeugt werden soll, ist das Gehäuse vorzugsweise zweiteilig und mit Scharnier- und Verschlußmitteln versehen.

Bei Verwendung axial gegenüberstehender Bereiche mit abwechselnder Polfolge können zweckmäßigerweise die Magnetfolien oder -bänder spiralförmig gewickelt sein. Auch auf diese Weise läßt sich eine Vergrößerung der Magnetfoliendicke und damit der Flußdichte erreichen.

Die für die Erfindung verwendeten Magnetfolien oder -bänder sind bevorzugt ein- oder beidseitig mit Textilmaterial kaschiert. Die Dicke der Magnetfolien oder -bänder kann im Bereich von 0,5—4,0 mm, vorzugsweise von 1,0—1,5 mm liegen.

Die Magnetfolien oder -bänder können auch innerhalb eines Textilbandes, z.B in Taschen, angeordnet sein, das an seinen freien Enden mit einem Verschluß, z.B. einem Klettverschluß, versehen ist. Diese Maßnahme erlaubt ein einfaches Anlegen der dauermagnetischen Anordnung um einen Gegenstand.

Eine weitere Vergrößerung der Flußdichte läßt sich dadurch erreichen, daß die Magnetfolien oder -bänder aus einem anisotropen Werkstoff bestehen.

Mit der dauermagnetischen Anordnung gemäß der Erfindung kann etwa die gleiche Kraftliniendichte wie bei elektromagnetisch erregten Spulen erreicht werden. Die erfindungsgemäße Anordnung ist jedoch wesentliche einfacher gestaltet und preiswürdiger. Sie kostet etwa nur ein Zehntel der derzeit auf dem Markt befindlichen Elektromagnetspulengeräte. Außerdem benötigt sie keinen elektrischen Stromanschluß, so daß keine Probleme hinsichtlich eines Kurzschlusses oder einer zu hohen Erwärmung auftreten können. Sie kann von jeder Person ohne Risiko auf einfache Art und Weise gehandhabt werden.

Nachfolgend sind Ausführungsbeispiele der Erfindung an Hand der Zeichnungen erläutert.

Es zeigen:

Fig. 1 ein Ausführungsbeispiel der dauermagnetischen Anordnung im senkrechten Längsschnitt in schmatischer Darstellung,

Fig. 2 einen senkrechten Querschnitt durch die Anordnung nach der Linie I—I der Fig. 1,

Fig. 3 den gleichen Querschnitt wie in Fig. 2, jedoch mit zwei schichtweise übereinander angeordneten Magnetfolien,

Fig. 4 ein Ausführungsbeispiel der Anordnung, bei der zwei rohrförmig ausgebildete Magnetbänder mehrlagig getrennt nebeneinander angeordnet sind,

Fig. 5 ein Ausführungsbeispiel, bei dem die getrennt angeordneten rohrförmigen Magnetbänder in einem starren Gehäuse untergebracht sind,

Fig. 6 ein Ausführungsbeispiel, bei dem die mehrlagige rohrförmige Magnetanordnung eine axial entgegensetzte Magnetisierung aufweist,

Fig. 7 und 8 je ein Anwendungsbeispiel, bei der die dauermagnetische Anordnung als Bandage ausgebildet ist im senkrechten Längsschnitt und in Aufsicht,

Fig. 9 die magnetische Bandage im therpeutischen Anwendungszustand im senkrechten Querschnitt.

Bei sämtlichen Ausführungsbeispielen ist die dauermagnetische Anordnung 1 aus einer oder mehreren flexiblen Magnetfolien zusammengesetzt. Diese Magnetfolien bestehen aus einem gummiartig-flexiblen, thermoplastischen Bindemittel, in das hochkoerzitive Dauermagnetpartikel in feiner Verteilung eingebettet sind. Die Mischung aus Dauermagnetwirkstoff und Bindemittel wird auf einem Kalander zu der folie in der benötigten Dicke geformt.

Als pulverförmige Dauermagnetwerkstoffe sind besonders isotrope oder anisotrope Ferrite auf der Basis von Barium- und/oder Strontiumferrit sowie Bleiferrit oder Kobalt-Seltenerdlegierungen sowie Neodymeisen geeignet. Letztere weisen bei sehr hoher Koerzitivkraft und Remanenz ein besonders hohes Energieprodukt auf.

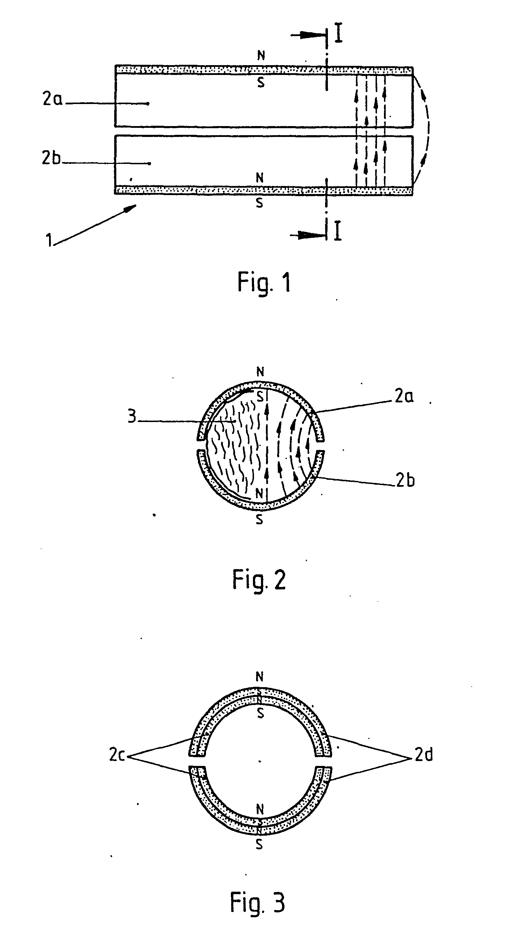

Die dauermagnetische Anordnung 1 gemäß der Erfindung ist in den Figuren 1—6 in schmatischer Darstellung gezeigt, um im angelegten Zustand an ein Körperteil die Magnetisierung und den magnetischen Feldlinienverlauf auf das Körperteil zu veranschlaulichen.

Wie aus den Figuren 1—6 ersichtlich ist, weist die Magnetfolie 2 der dauermagnetischen Anordnung 1 eine rohrförmige Gestalt auf. Bei dem Ausführungsbeispiel gemäß Fig. 1 und 2 besteht die Magnetfolie aus zwei Magnetfolienteilen 2a und 2b, von denen jedes während der therapeutischen Anwendung eine halbrohrförmige Gestalt aufweist. Zwischen den Folienteilen befindet sich das Körperteil 3. Die Befestigungsteile sind in der Zeichnung nicht dargestellt.

Die Magnetfolie bzw. -folienteile 2a und 2b weisen eine Magnetisierungsrichtung auf, die senkrecht zur Folienoberfläche verläuft. Hierbei sind die Folientiele 2a und 2b so zueinander angeordnet, daß die innere Fläche des Folienteils 2a die dem Körperteil zugekehrt ist, einen Südpol und die innere Fläche des gegenüberliegenden Folienteils 2b einen Nordpol besitzt. Durch diese besondere Anordnung ergibt sich ein erwünschter axialer Feldlinien verlauf vom Nordpol zum Südpol im Bezug auf die röhrenförmige Anordnung im Innern dieser Anordnung.

Dieser Verlauf der magnetischen Feldlinien ist in den Fig. 1 und 2 durch gestrichelte Linien dargestellt. Die Pole sind durch die Buchstaben N und S gekennzeichnet. Wie man aus den Figuren erkennt, liegen die entsprechenden Gegenpole auf den Außenflächen der Folien bzw. Folienteile.

In Fig. 3 ist die rohrförmige dauermagnetische Anordnung mit der gleichen Polanordnung wie in den Fig. 1 und 2 dargestellt. Bei diesem Ausführungsbeispiel sind jedoch jeweils zwei dünne Magnetfolienteile 2c, 2d mit gleicher Dicke schichtweise übereinander angeordnet, wobei jeweils ein Nordpol der einen folie dem Südpole der benachbarten Folie gegenüberliegt. Die Polfolge ist in der Zeichnung wiederum durch die Buchstaben N und S gekennzeichnet. Bei dieser Anordnung wird der Vorteil erreicht, daß bei etwa gleichbleibender Flexibilität die wirksame Feldliniendichte verdoppelt wird.

Bei der vorbeschriebenen Anordnung mit diametral verlaufenden Feldlinien wurde bei einem Durchmesser des Innenraumes der dauermagnetischen Anordnung, der etwa dem Durchmesser des Körperteils entspricht, bei isotropen Magnetfolien von 3,0 mm Dicke eine magnetische Feldliniendichte von 13 mT (130 G) gemessen.

Es liegt im Rahmen der Erfindung, auch mehr als zwei dünne Folien übereinander zu schichten. Dies ist besonders erforderlich, wenn bei großem Durchmesser (etwa bei Anwendung um den Oberschenkel) eine sehr hohe Feldliniendichte verlangt wird. Die Gesamtdicke der geschichteten Anordnung kann hier sogar ca. 10—15 mm betragen.

Diese Art der Vergrößerung der Foliendicke ist besonders auch bei wohlfeilen Magnetmaterialien, wie z.B. isotropes bariumferrit, vorteilhaft.

Eine Verstärkung der wirksamen Feldliniendichte wird auch erreicht, wenn die Magnetanordnung aus anisotropen Magnetfolien besteht.

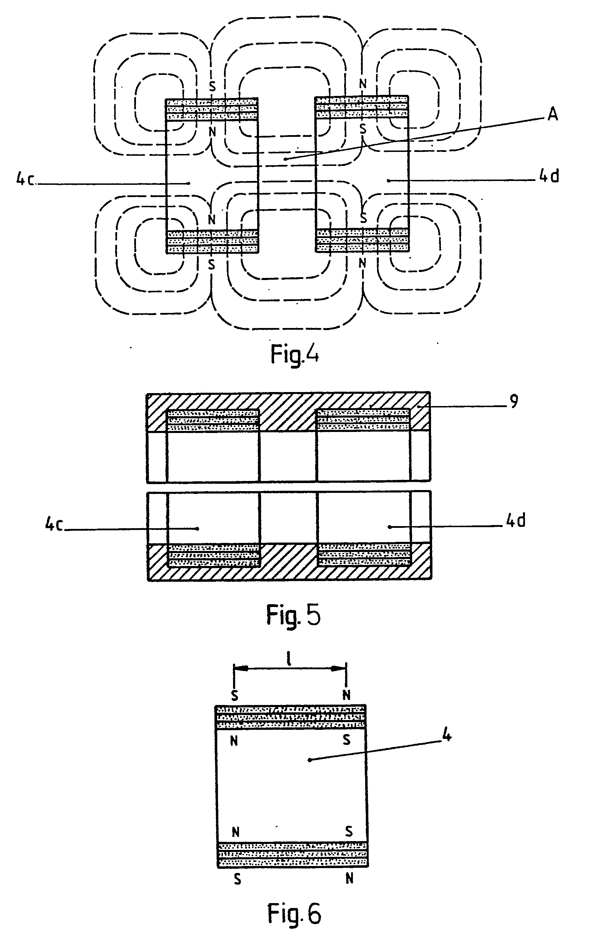

Bei der magnetichen Anordnung gemäß Fig. 4 sind zwei Magnetfolien oder -bänder 4c, 4d rohrförmiger Gestalt getrennt in axialer Richtung nebeneinander angeordnet. Das eine rohrförmig gestaltete Magnetband 4c besitzt an der nach innen weisenden Seite einen umlaufenden Nordpol und das andere rohrförmige Magnetband 4d an der nach innen weisenden Seite einen umlaufenden Südpol, wie in der Zeichnung durch die Buchstaben N und S gekennzeichnet. Bei diesem Beispiel sind zwecks Vergrößerung der Magnetbanddicke bzw. der Feldliniendichte drei Magnetbänder schichtweise übereinander angeordnet. Bei dieser Ausführung wird im Bereich A eine verhältnismäßig gute Feldhomogenität erzielt.

Gemäß Figur 5 sind die getrennt nebeneinander angeordneten Magnetfolien oder -bänder 4c und 4d rohrförmiger Gestalt in einem vorzugsweise zweiteiligen Gehäuse 9 angebracht. Die Scharnier- und Verschlußmittel sind in der Zeichnung nicht dargestellt.

Man kann, wie in Fig. 6 dargestellt ist, die Magnetfolie 4 auch zweipolig mit senkrecht zur Folienoberfläche verlaufender Magnetisierungsrichtung aufmagnetisieren, derart, daß auf der nach innen weisenden Seite des rohrförmigen Gebildes wenigstens ein umlaufender Nordpol und wenigstens ein umlaufender Südpol mit großem Abstand nebeneinander vorhanden sind. Die Pole sind wiederum mit den Buchstaben N und S gekennzeichnet.

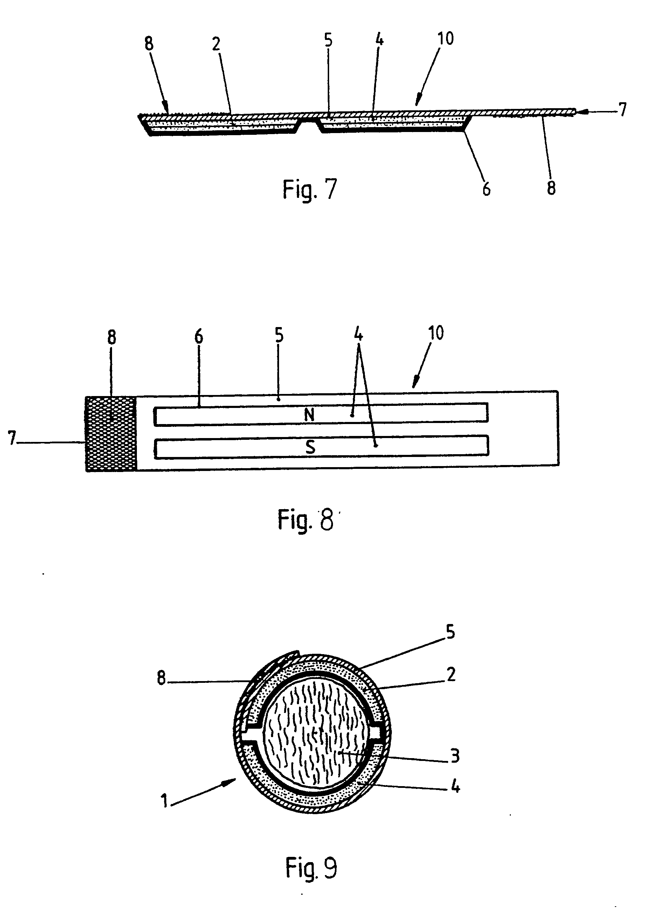

In den Fig. 7 und 8 ist die dauermagnetische Anordnung beispielsweise in Form einer Bandage 10 im nicht angelegten Zustand dargestellt. In einem Textilgewebeband 5 gemäß Fig. 7 sind je zwei Magnetfolienteile 2, 4 im Abstand zueinander, z.B. in Taschen 6, angeordnet. Die Magnetfolienteile können entweder eine Magnetisierung aufweisen, wie sie in den Fig. 1—3 oder 4 dargestellt ist. Sie können auch in mehreren Lagen übereinander angeordnet sein. Die freien Enden 7 des Textilgewebebandes 5 sind mit einem Klettverschluß 8 versehen. Es ist jedoch auch eine andere Verschlußart denkbar.

Bei der in Fig. 8 dargestellten Bandage 10 sind zwei Magnetbänder 4 getrennt voneinander und parallel nebeneinander vorzugsweise in Taschen 6 eines Textilgewebebandes 5 eingelegt. Wie aus der Zeichnung ersichtlich ist, befindet sich auf dem einen Magnetband ein Nordpol auf der nach innen weisenden Oberfläche und auf dem benachbart liegenden Magnetband ein Südpol.

Es können bei dieser Anordnung auch mehrere Magnetbänder übereinander in je einer Tasche lose und daher gegeneinander in Längsrichtung verschiebbar angeordnet sein. Die freien Enden 7 des Textilgewebebandes 5 sind wiederum mit einem Klettverschluß 8 versehen.

Die in den Fig. 7 und 8 dargestellte Bandage ist gemäß Fig. 9 um ein Körperteil 3, z.b. einen Arm, gelegt. Man erkennt die rohrförmige Gestalt der Magnetfolienteile 2, 4, die das Körperteil vollständig umschließen. Entsprechend der erfindungsgemäß vorgeschlagenen Magnetpolanordnungen durchdringen die magnetischen Feldlinien das Körperteil 3 in Längs- und/oder Querrichtung.

Bei der in Fig. 8 dargestellten Bandage 10 besitzen die beiden Magnetbänder 4 im angelegten Zustand um ein Körperteil 3 gemäß Fig. 9 eine rohrförmige bzw. ringförmige Gestalt.

Die Erfindung ist nicht auf die hier dargestellten Ausführungsbeispiele beschränkt. Vielmehr ist es möglich, die Magnetfolie oder -bänder in Kleidungsstücke einzunähen oder auf ihnen zu tragen, z.B. auf dem Strumpf am Bein. Infolge der großen Eindringtiefe der magnetischen Feldlinien kann in diesen Fällen das erwünschte magnetostatische Feld auch in dem betreffenden Körperteil erzeugt werden.

**Patentansprüche**

1. Dauermagnetische Anordnung, die aus einer oder mehreren gummiartig-flexiblen Magnetfolien oder -bändern mit darin eingebetteten hochkoerzitiven dauermagnetischen Teilchen besteht, die eine röhrenförmige Gestalt besitzt oder in eine derartige Form gebracht werden kann und die eine senkrecht zur Folien- bzw. Bandoberfläche verlaufende Magnetisierung aufweist, dadurch gekennzeichnet, daß die gegensätzlichen Magnetpole (S, N) sich diametral oder axial in Bezug auf die röhrenförmige Gestalt gegenüberstehen und der Gesamtinduktionsfluß des oder der Südpole (S) an der einen, nach innen weisenden Seite der Anordnung gleiche dem Gesamtinduktionsfluß des oder der Nordpole (N) an der anderen, nach innen weisenden Seite der Anordnung ist, und etwa eine homogene Kraftliniendichte erzeugen.

2. Dauermagnetische Anordnung nach Anspruch 1, gekennzeichnet durch zwei sich diametral gegenüberstehende halbröhrenförmige Anordnungen (Magnetfolienteile 2a und 2b), wobei jede Anordnung eine oder mehrere übereinander angeordnete Magnetfolien oder -bänder (Magnetfolienteile 2c und 2d) aufwiest und wobei die nach innen weisenden Seiten der Magnetfolien oder -bänder (Magnetfolienteile 2c und 2d) der einen Anordnung eine bestimmte Polarität und die nach innen weisenden Seiten der anderen Anordnung die dazu entgegengesetzte Polarität aufweisen.

3. Dauermagnetische Anordnung nach Anspruch 1, gekennzeichnet durch mindestens zwei sich axial gegenüberstehende röhrenförmige Bereiche mit abwechselnder Polfolge.

4. Dauermagnetische Anordnung nach Anspruch 3, dadurch gekennzeichnet, daß die Bereiche durch unterschiedliche Magnetisierungen einer oder mehrerer übereinander angeordneter Magnetfolien oder -bänder gebildet sind.

5. Dauermagnetische Anordnung nach Anspruch 4, dadurch gekennzeichnet, daß die Bereiche durch getrennt nebeinander angeordnete Magnetfolien oder -bänder (4c, 4d) gebildet sind.

6. Dauermagnetische Anordnung nach Anspruch 5, dadurch gekennzeichnet, daß die getrennt nebeneinander angeordneten Magnetfolien oder -bänder (4c, 4d) in einem starren Gehäuse (9) angebracht sind.

7. Dauermagnetische Anordnung nach Anspruch 6, dadurch gekennzeichnet, daß das Gehäuse (9) zweiteilig und mit Scharnier- und Verschlußmitteln versehen ist.

8. Dauermagnetische Anordnung nach einem der Ansprüche 4 bis 7, dadurch gekennzeichnet, daß die Magnetfolien oder -bänder spiralförmig gewickelt sind.

9. Dauermagnetische Anordnung nach einem der Ansprüche 1 bis 8, dadurch gekennzeichnet, daß die Magnetfolien oder -bänder ein- oder beidseitig mit Textilmaterial kaschiert sind.

10. Dauermagnetische Anordnung nach einem der Ansprüche 1 bis 9, dadurch gekennzeichnet, daß die Magnetfolien oder -bänder eine Dicke von 0,5—4,0 mm, vorzugsweise von 1,0—1,5 mm haben.

11. Dauermagnetische Anordnung nach einem der Ansprüche 1 bis 10, dadurch gekennzeichnet, daß die Magnetfolien oder -bänder (2, 4) innerhalb eines Textilbandes (5), z.B. in Taschen (6), angeordnet sind, das an seinen freien Enden (7) mit einem Verschluß (Klettverschluß 8) versehen ist.

12. Dauermagnetische Anordnung nach einem der Ansprüche 1 bis 11, dadurch gekennzeichnet, daß die Magnetfolien oder -bänder aus einem anisotropen Werkstoff bestehen.

**Revendications**

1. Dispositif magnétique permanent, qui se compose d'une ou de plusieurs feuilles ou bandes magnétiques ayant la flexibilité du caoutchouc, auxquelles sont incorporées des particules magnétiques permanentes de haute coercivité, et qui a une forme tubulaire ou peut être mit sous une telle forme et qui présente une aimantation dirigée perpendiculairement à la surface des feuilles ou des bandes, caractérisé en ce que les pôles magnétiques contraires (S, N) sont opposés diamétralement ou axialement par rapport à la forme tubulaire, en ce que le flux d'induction total du ou des pôles Sud (s) à travers l'une des faces, dirigée vers l'intérieur, du dispositif est égal au flux d'induction total du ou des pôles Nord (N) à travers l'autre face dirigée vers l'intérieur du dispositif, et en ce que ces flux produisent une densité magnétique à peu près homogène.

2. Dispositif magnétique permanent selon la revendication 1, caractérisé par deux dispositifs demitubulaires diamétralement opposés (éléments en feuille magnétique 2a et 2b), chaque dispositif comportant une ou plusieurs feuilles ou bandes magnétiques superposées (éléments en feuille magnétique 2c et 2d) et les faces dirigées vers l'intérieur des feuilles ou bandes magnétiques (éléments en feuille magnétique 2c et 2d) de l'un des dispositifs présentant une polarité déterminée et les faces dirigées vers l'intérieur de l'autre dispositif présentant la polarité contraire à celle-ci.

3. Dispositif magnétique permanent selon la revendication 1, caractérisé par au moins deux régions tubulaires opposées axialement à succession alternante des pôles.

4. Dispositif magnétique permanent selon la revendication 3, caractérisé en ce que lesdites régions sont formées par des aimantations différentes d'une ou de plusieurs feuilles ou bandes magnétiques superposées.

5. Dispositif magnétique permanent selon la revendication 4, caractérisé en ce que lesdits régions sont formées par des feuilles ou bandes magnétiques (4c, 4d) placées l'une à côté de l'autre en position écartée.

6. Dispositif magnétique permanent selon la revendication 5, caractérisé en ce que les feuilles ou bandes magnétiques (4c, 4d) placées l'une à côté de l'autre en position écartée sont disposées dans une enveloppe rigide (9).

7. Dispositif magnétique permanent selon la revendication 6, caractérisé en ce que l'enveloppe (9) est en deux parties et est minue de moyens à charnière et de moyens de fermeture.

8. Dispositif magnétique permanent selon l'une quelconque des revendications 4 à 7, caractérisé en ce que les feuilles ou bandes magnétiques sont enroulées en spirale.

9. Dispositif magnétique permanent selon l'une quelconque des revendications 1 à 8, caractérisé en ce que les feuilles ou bandes magnétiques sont doublées de matière textile sur une face ou sur les deux faces.

10. Dispositif magnétique permanent selon l'une quelconque des revendications 1 à 9, caractérisé en ce que les feuilles ou bandes magnétiques ont une épaisseur de 0,5 à 4,0 mm, de préférence de 1,0 à 1,5 mm.

11. Dispositif magnétique permanent selon l'une quelconque des revendications 1 à 10, caractérisé en ce que les feuilles ou bandes magnétiques (2, 4) sont disposées à l'intérieur d'une bande textile (5), par exemple dans des poches (6), cette bande étant munie d'une fermeture (fermeture à accrochage 8) à ses extrémités libres.

12. Dispositif magnétique permanent selon l'une quelconque des revendications 1 à 11, caractérisé en ce que les feuilles ou bandes magnétiques sont faites d'une matière anisotrope.

**Claims**

1. Permanent magnet arrangement, which consists of one or a plurality of magnetic foils or magnetic tapes, flexible in the manner of rubber, with highly coercive permanent magnetic particles embedded therein, which possesses a tubular shape or can be brought into such a form, and has a magnetization extending perpendicular to the foil surface or tape surface, characterized in that the opposite magnet poles (S, N) are diametrically or axially opposed in relation to the tubular shape, and the total magnetic flux of the south pole or south poles (S) at one, inwardly directed side of the arrangement is equal to the total magnetic flux of the north pole or north poles (N) at the other, inwardly directed side of the arrangement, and generates approximately a homogeneous force line density.

2. Permanent magnet arrangement according to Claim 1, characterized by two diametrically opposed semi-tubular arrangements (magnetic foil parts 2a and 2b), each arrangement having one or a plurality of arranged magnetic foils or magnetic tapes (magnetic foil parts 2c and 2d), and the inwardly directed sides of the magnetic foils or magnetic tapes (magnetic foil parts 2c and 2d) of one arrangement having a specific polarity, and the inwardly directed sides of the other arrangement having the opposite polarity.

3. Permanent magnet arrangement according to Claim 1, characterized by at least two axially opposed tubular regions with alternating pole sequence.

4. Permanent magnet arrangement according to Claim 3, characterized in that the regions are formed by different magnetizations of one or a plurality of magnetic foils or magnetic tapes arranged one above another.

5. Permanent magnet arrangement according to Claim 4, characterized in that the regions are formed by magnetic foils or magnetic tapes (4c, 4d) arranged separately next to one another.

6. Permanent magnet arrangement according to Claim 5, characterized in that the magnetic foils or magnetic tapes (4c, 4d) arranged separately next to one another are fitted in a rigid housing (9).

7. Permanent magnet arrangement according to Claim 6, characterized in that the housing (9) is made in two parts and provided with hinging means and closing means.

8. Permanent magnet arrangement according to one of Claims 4 to 7, characterized in that the magnetic foils or magnetic tapes are helically wound.

9. Permanent magnet arrangement according to one of Claims 1 to 8, characterized in that the magnetic foils or magnetic tapes are lined with textile material on one or both sides.

10. Permanent magnet arrangement according to one of Claims 1 to 9, characterized in that the magnetic foils or magnetic tapes have a thickness of 0.5—4.0 mm, preferably of 1.0—1.5 mm.

11. Permanent magnet arrangement according to one of Claims 1 to 10, characterized in that the magnetic foils or magnetic tapes (2, 4) are arranged inside a textile tape (5), e.g. in pockets (6), which textile tape is provided at its free ends (7) with a closure (burr closure 8).

12. Permanent magnet arrangement according to one of Claims 1 to 11, characterized in that the magnetic foils or magnetic tapes consists of an anisotropic material.

EP 0 198 958 B1

Fig. 1

Fig. 2

Fig. 3

Fig.4

Fig. 5

Fig. 6

Fig. 7

Fig. 8

Fig. 9